# EUROPEAN PATENT APPLICATION

(11) **EP 4 151 274 A1**
(43) Date of publication of application: **22.03.2023**
(21) Application number: 20935802.7
(22) Date of filing: 20.05.2020
(51) Int. Cl.: A61N 5/02

(54) **GLOBAL HYPERTHERMIA SYSTEM COMBINING MICROWAVE AND RADIOFREQUENCY ROTARY RADIATION**

(30) Priority: 13.05.2020 CN 202010403212
(71) Applicant: Shang, Shengjie, Jieshou, Anhui 236500 (CN)
(72) Inventor: SHANG, Zhiying, Anhui 236500 (CN); SHANG, Chengde, Anhui 236500 (CN); SHANG, Shengjie, Anhui 236500 (CN)
(74) Representative: Vitina, Maruta
(86) International application number: PCT/CN2020/091361
(87) International publication number: WO 2021/227113

(57) **Abstract**

A global irradiation thermotherapy system based on coordinated rotation of microwave and radio frequency, including an intelligent control unit, a microwave rotary heating mechanism, a capacitive radio frequency rotary heating mechanism and a temperature-measuring mechanism connected with the intelligent control unit. The intelligent control unit coordinately controls the microwave rotary heating mechanism and the capacitive radio frequency rotary heating mechanism to rotationally irradiate a thermotherapy area to perform deep-shallow layered and complementary diathermy to achieve uniform heating of all tissues in the thermotherapy area. The temperature measuring mechanism obtains precise temperature data of the thermotherapy area of the human body via optical-fiber temperature-measuring technology as a reference system, and accurately measures a temperature of each point in the thermotherapy area based on combination of nuclear magnetic thermal imaging technology, so as to achieve precise temperature control and achieve uniform and precise hyperthermia throughout the thermotherapy area.

## Description

### TECHNICAL FIELD

The present disclosure relates to medical techniques, in particular to a global irradiation thermotherapy system based on coordinated rotation of microwave and radio frequency.

### BACKGROUND

Currently, surgery, chemotherapy, and radiotherapy are considered as the most conventional methods for the clinical treatment of cancer. They alone or in combination have good curative effects on the early radical treatment of tumors that have not experienced metastasis, but they are often less effective for the treatment of middle and advanced tumors, and thus fail to prevent the metastasis and spread. Therefore, scientific researchers in the medical field all over the world have been committed to finding and exploring new treatment approaches for a long time. It is recorded in history that the German doctor Busch concerned in 1866 that one of his cancer patients had not received any anti-cancer treatment, but the high fever caused by erysipelas infection accidentally caused the cancer to disappear, and for the first time proposed the thermotherapy theory that high fever can cure cancer. In 1893, Coley published his research on "febrile therapy" in the American Journal of Medicine. He experimentally injected tumor patients with a mixed extract of Streptococcus pyogenes and Pseudomonas aeruginosa (that is erysipelas toxin, also known as Coley toxin) to induce the patient to have a fever of 38.0-40.2°C, 38 patients with advanced malignant tumors, of which 12 cases completely resolved, 19 cases improved, and the 5-year survival rate reached 60%. In 1918, Robdendury reported that among the collected 166 cases of malignant tumors that resolved spontaneously without any treatment, among them, 72 cases were found to be accompanied by severe infection and high fever. In 1957, Selawry synthesized 450 cases of malignant tumors that had spontaneously resolved through various documents and found that at least 150 of them had a history of malaria and typhoid fever that induced high fever. In the 1980s, Ishihara, a Japanese medical doctor, published "Body Temperature Determines Birth, Old Age, Sickness and Death", proposing the idea that a temperature exceeding 39°C can scald cancer cells to death. In 1985, the FDA (U.S. Food and Drug Administration) certified that thermotherapy is the fifth largest tumor treatment after surgery, radiotherapy, chemotherapy, and biological therapy, and pointed out that thermotherapy is a green therapy! Since then, many countries have actively carried out research and adopted various heating methods to verify the theory of thermotherapy. The successive results have all proved that thermotherapy is a rare method for the treatment of cancer, and the development of thermotherapy equipment has a good prospect.

The inventor of the present invention comprehensively believes that in order to realize the radical cure of tumors, clarify the mechanism of thermotherapy treatment, search for global thermotherapy technology, optimize the best temperature range for high fever and blanching, and ensure accurate temperature control have become the four major technical problems.

The inventors have also conducted in-depth research on cancer cells and found that due to the rapid differentiation of genetic mutations, cancer cells grow out of control, and can proliferate indefinitely. Under normal circumstances, they need to absorb a large amount of nutrients from the outside world and input excessive oxygen. At this time, the stress response is that respiration will rapidly intensify. Due to mutations such as tissue vascular disorder and obstruction due to cancer cell gene mutations, it is difficult to adapt to the excessive demand for oxygen by cancer cells under high fever. As a result, it will cause anaerobic respiratory stress, and a large amount of lactic acid produced by anaerobic accumulation will not have time to efflux, leading to acidification of cancer cell tissues. These acidified substances cause fatal harm to cancer cell tissues: acidic environment will inhibit cancer cell protein and chromosome replication and cause proliferation failure. At this time, cancer cells can continue surviving and not being scalded to death, but no longer differentiated and proliferated, which is equivalent to losing the replication function, and falling into a state of apoptosis. This inactive cancer cell is then eliminated through two ways: one is that one by one will continue to die with the metabolism, secondly, because of its acidic characteristics, it is easier to be recognized and eliminated by immune cells such as lymph. Therefore, the impact of high fever and blanch on cancer cells is an irreversible killing. One or more thermotherapy can achieve a radical cure. In the human body tolerable temperature range (within 47°C), the higher the temperature, the stronger the anaerobic respiratory stress of cancer cells, and the more thorough the apoptosis caused by the acidification of cancer cell tissues. The high fever over 42°C will cause cancer cells to die if they are blanched for more than 30 minutes, the mortality rate is more than 90%, but if the temperature exceeds 43°C, the high fever will be more and more harmful to the normal cells of the human body. Therefore, the best temperature range for high-temperature blanching should be 42-43°C, and the highest limit should not exceed 43°C. Cancer cells and normal human cells exhibit significant difference at 43°C, specifically, the cancer cells become inactivated while the normal cells are less damaged, which makes this temperature possible for thermotherapy to cure tumors.

In-depth studies have found that although the current various tumor thermotherapy methods have certain feasibility, they also have various problems and shortcomings, which affect the final curative effect to varying degrees, making it difficult for the existing thermotherapy techniques to cure the tumor. In order to facilitate the improvement of the process and the development of more scientific and reasonable thermotherapy technology, we have carried out a systematic analysis and research on various existing thermotherapy methods, found out the crux of the problem, and proposed improvement ideas.

At present, thermotherapy methods are divided into two categories: one is non-equipment thermotherapy methods, which are mostly based on the most primitive cases of measles or malaria infection with high fever to treat cancer, and inject "toxin" into cancer patients to induce infection and fever, so as to burn the cancer cells. These methods have definite results, but the risks are difficult to control and cannot be promoted. The other is equipment-based thermotherapy, which uses equipment to heat to kill cancer cells. According to different heating methods, the equipment-based thermotherapy methods are divided into the following types.

One is the space capsule tumor thermotherapy system, which allows patients to be placed in the space capsule to perform thermotherapy with infrared rays or high-temperature steam. Although this is a global heating idea, because the human body has the function of self-defense, it resists the outside high temperature through sweating to guarantee a constant temperature inside the human body, so infrared or high-temperature steam has a poor effect on the human body, and it is difficult for the deep part of the human body to reach high temperature. This equipment is only effective for shallow tumors that have not metastasized.

The second is a tumor microwave thermotherapy device. Theoretically, the following four characteristics of microwave are very suitable for making a tumor thermotherapy instrument: (1) the heating penetration is good: the inside and outside of the medium can be heated uniformly at the same time; (2) select heating: only for the water-containing tissue generates heat, but almost penetrates through the tissues with low water content such as fat and protein without generating heat; (3) the thermal inertia is small, and it stops at once, which is conducive to precise temperature control; (4) non-ionizing: the effect of microwave on the human body is only physical heating effect, no chemical hazard. However, from the actual application of the N-9001 microwave tumor therapy instrument (915 MHz) developed by Jiangsu Nowan Medical Equipment Co. Ltd. and the UNI-3000 microwave thermotherapy device (2450 MHz) developed by Hunan Youli Medical Technology Co. Ltd., there are some problems with microwave thermotherapy. Technical defects, in-depth research found that the root causes are: first, the microwave penetration depth is not enough, usually no more than 6cm (including fat thickness), the directional heating coverage is very small, and it is limited to the treatment of superficial tumors that have not metastasized.

The third is the tumor radio frequency thermotherapy device, which is a new type of electromagnetic thermotherapy device based on the research and improvement of microwave thermotherapy technology. Although it has broken through the limitation of the depth of diathermy, the application effect is still not optimistic, and it is difficult to radically cure the spread and metastasis tumor. Through in-depth research on various radio frequency thermotherapy devices such as Jilin Maida NRL-003 (30-40 MHz difference frequency), Japan FR-8 (8 MHz), American BSD-2000W (60-120 MHz), etc., it is found that there are two common problems in all these radio frequency thermotherapy devices. Firstly, radio frequency heating tends to cause overheating of the superficial fat layer, which must be laminated with a water bag for cooling treatment. Secondly, the current radio frequency thermotherapy device adopts multiple sets of capacitive radio frequency rotary heating mechanisms to directional cross radiation to strengthen the heating and scald the found tumor lesions, and lacks global thermotherapy thinking and radical cure concepts.

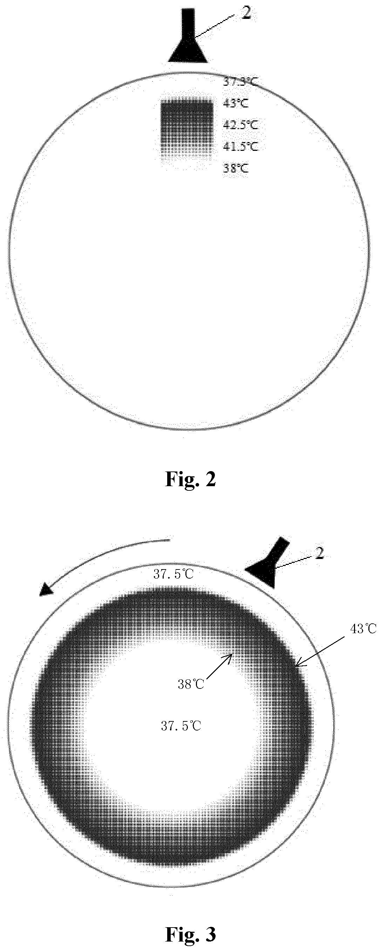

Chinese Patent Publication No. 106823152A, titled "A Cancer Therapeutic Apparatus" and Chinese Patent Publication No. 110694178A, titled "A Tumor Horizontal Microwave Global Thermotherapy Device", both disclosed a global treatment device, but due to the insufficient heat penetration depth of a single microwave thermotherapy, even if the rotary heating method is used, only a ring-shaped high temperature layer with the thickness no more than 4cm can be formed inside the fat layer, and the temperature gradually decreases from the outside to the inside. It can kill non-metastatic tumors located in the superficial layer, and cannot be used for deep tumor treatment. The comparison of the effects of microwave static directional heating and microwave rotary heating is shown in Fig. 2 and Fig. 3.

In addition, field investigations also found that today's human body internal temperature measurement technology has shortcomings. Various thermotherapy devices are not equipped with accurate internal temperature measurement instruments. Thermotherapy cannot accurately control the temperature. For safety reasons, it is forced to lower the temperature at the expense of the effect of thermotherapy. Conservative irradiation also affects the real effect of thermotherapy equipment, so current thermotherapy devices have not achieved the expected results.

At present, there are only two types of temperature measurement that are suitable for anti-electromagnetic interference in the body. One is the optical fiber method, which can accurately measure the temperature of certain positions in the body through invasive implantation. Although it is not subject to electromagnetic interference, it can be used for multi-point temperature measurement in the body. However, invasive implantation not only increases the patient's pain and infection risk, but is also restricted by many factors. It cannot reach any part of the human body (such as the liver, lungs, kidneys, and other organs) at will, so it is impossible to obtain the temperature information of all the location in internal parts of the human body. Another type of nuclear magnetic thermal imaging method uses nuclear magnetic thermal technology combined with big data to calculate through software. Although it can obtain the human body temperature information non-invasively, the temperature measurement error of this method is relatively large, which is not conducive to precise temperature control.

In-depth research has found that the temperature measurement errors of nuclear magnetic thermal imaging technology are mostly caused by changes in environmental factors such as voltage, humidity, magnetic field and host operating temperature. The errors caused by changes in these factors have the same deviation characteristics in the same direction. This discovery is based on the use of optical fibers. The temperature measurement technology has found a basis for correcting the temperature measurement error of nuclear magnetic thermal imaging, and is also conducive to the invention of a method for accurate and non-destructive temperature measurement in the body.

Based on the above analysis, there is an urgent need to provide a new type of microwave radio frequency coordinated rotation global radiation thermal therapy system and its device to solve the above-mentioned problem of global thermal therapy under precise temperature control.

### SUMMARY

The technical problem to be solved by the present disclosure is to provide a global irradiation thermotherapy system based on coordinated rotation of microwave and radio frequency, which utilize microwave and radio frequency rotation irradiation combined with precise temperature measurement technology to realize the whole area of the human body with precise high heat and blanching.

In order to solve the above technical problems, the first technical solution adopted by the present disclosure is to provide a global irradiation thermotherapy system based on coordinated rotation of microwave and radio frequency, comprising:
an intelligent control unit;
a microwave rotary heating mechanism;
a capacitive radio frequency rotary heating mechanism; and
a temperature measuring mechanism;
wherein the microwave rotary heating mechanism, the capacitive radio frequency rotary heating mechanism and the temperature measuring mechanism are connected to the intelligent control unit;
the capacitive radio frequency rotary heating mechanism is configured to rotationally heat each point in an area of a human body with a lateral radius of 0-13 cm through capacitive radio frequency rotary irradiation to form a circular high-temperature zone, wherein a temperature of the circular high-temperature zone decreases from center to outside;
the microwave rotary heating mechanism is configured to form an annular high-temperature layer with a thickness of 2-4 cm on an inner side of a fat tissue through rotary microwave irradiation, and a temperature of the high-temperature layer decreases from outside to inside;
the temperature measurement mechanism is configured to obtain precise temperature data of several key points in an accessible part of a thermotherapy area of the human body via optical-fiber temperature-measuring technology as a reference system, and to accurately measure a temperature of each point in the thermotherapy area based on combination of nuclear magnetic thermal imaging technology; and
the intelligent control unit is configured to coordinately control the microwave rotary heating mechanism and the capacitive radio frequency rotary heating mechanism to rotationally irradiate the thermotherapy area to perform layered complementary diathermy, and to perform precise temperature control based on real-time temperature measurement of the temperature measuring mechanism to achieve uniform and precise hyperthermia throughout the thermotherapy area.

In an embodiment, a microwave frequency of the microwave rotary heating mechanism is 300 MHz-3000 MHz, and a radio frequency of the capacitive radio frequency rotary heating mechanism is 1 MHz-300 MHz.

In an embodiment, the temperature measuring mechanism comprises a nuclear magnetic thermal imaging device, an optical fiber temperature measuring device, and a data processing platform;
the nuclear magnetic thermal imaging device is configured to establish a temperature field distribution map library of hot points respectively corresponding to individual temperatures according to nuclear magnetic resonance scanning data, and to obtain a temperature field distribution map of the thermotherapy area of the human body by scanning;
the optical fiber temperature measuring device is configured to obtain the temperatures of several key points in the accessible part of the thermotherapy area as temperature calibration points by using a non-invasive technology;
the data processing platform is configured to calculate the temperature field distribution map scanned by the nuclear magnetic thermal imaging device based on the temperature field distribution map library to obtain a temperature scanning data of each hot point in the thermotherapy area, and is configured to calibrate the temperature scanning data of each hot point using temperature data of the temperature calibration points as reference to obtain accurate temperature data of each hot point in the thermotherapy area.

In an embodiment, the optical fiber temperature measurement device comprises a temperature measuring device comprises a temperature data acquisition unit, a temperature data processing unit and a sensing optical fiber; and a plurality of optical fiber sensors are provided on the sensing optical fiber.

In an embodiment, the global irradiation thermotherapy system further comprises a translational heat-preservation bin, wherein the translational heat-preservation bin is configured to carry the human body in and out of an internal space of the microwave rotary heating mechanism and the capacitive radio frequency rotary heating mechanism.

In a second aspect, the disclosure provides a method for measuring a temperature of a thermotherapy area of a human body using the global irradiation thermotherapy system, comprising:
(S401) scanning hot points using the nuclear magnetic thermal imaging device to obtain thermal imaging data, and establishing a temperature field distribution map library of the hot points respectively corresponding to individual temperatures;
(S402) obtaining the temperature field distribution map of the thermotherapy area of the human body by scanning using the nuclear magnetic thermal imaging device;
(S403) obtaining, by the optical fiber temperature measuring device, temperatures of several key points in the accessible part of the thermotherapy area as temperature calibration points by using a non-invasive technology; and
(S404) calculating the temperature field distribution map obtained in step (S402) based on the temperature field distribution map library obtained in step (S401) to obtain a temperature scanning data of each point in the thermotherapy area; and calibrating the temperature scanning data of each point in the thermotherapy area using temperature data of the temperature calibration points as a reference to obtain accurate temperature data of each point in the thermotherapy area.

In an embodiment, in step (S401), the temperature field distribution map library comprises thermal imaging data and temperature value of each hot point, and the thermal imaging data and temperature value of each hot point are in one-to-one correspondence.

In an embodiment, in step (S404), the temperature data of the temperature calibration points comprises accurate temperature value and location information; and the temperature field distribution map comprises thermal imaging information and location information of each point in the thermotherapy area; and the temperature calibration points have corresponding points in the temperature field distribution map.

Compared to the prior art, the present disclosure has the following beneficial effects.
(1) The present disclosure uses microwave combined with capacitive radio frequency rotary irradiation to uniformly heat both internal and external. It has a global hot-scalding thinking, fully simulating the human body's high fever and killing cancer cells, and achieves accurate temperature measurement through nuclear magnetic temperature measurement with the help of optical fiber technology, ensuring accurate control of high fever and blanching, stable and controllable heating, and patients can also withstand systemic and local treatments, with good therapeutic effects, no damage to healthy tissues, and no adverse side effects.
(2) The present disclosure takes advantage of the deep heating of radio frequency and adopts the rotary heating method to strengthen the scanning and heating of each point in the human body with a lateral depth of more than 6 cm, avoiding the problem of overheating of the surface fat layer caused by radio frequency radiation, and enabling the deep layer to be focused to achieve high temperature.
(3) The present disclosure uses the feature of microwave selective heating to avoid the superficial fat layer, to ensure that the fat will not overheat, and to compensate the defect that the 2-6 cm middle layer is not enough due to the insufficient heat of the radio frequency rotary radiation to realize the overall uniform heating.
(4) The present disclosure combines the nuclear magnetic resonance thermal imaging technology with the optical-fiber temperature-measuring technology, which not only realizes the non-invasive measurement of the wide-area temperature in the body, greatly reduces the pain of the patient, but also realizes accurate temperature measurement and realizes the precise control of thermotherapy temperature, ensures the safety and efficiency of thermotherapy.
(5) The system and the global thermotherapy device of the present disclosure adopt pure physical precision temperature control thermotherapy, which is safe, convenient and efficient, and is applied in the field of tumor treatment, which can cure various solid tumors and keep normal tissues and organs intact. It has the characteristics of short time, less pain and low cost for tumor treatment, and has a wide range of indications.

### BRIEF DESCRIPTION OF THE DRAWINGS

Fig. 1 is a structural block diagram of the microwave radio frequency coordinated rotation global radiation thermotherapy system of the present disclosure;
Fig. 2 is a schematic diagram of the effect of microwave static directional heating;
Fig. 3 is a schematic diagram of the effect of microwave rotation heating;
Fig. 4 is a schematic diagram of radio frequency static unidirectional heating effect.
Fig. 5 is a schematic diagram of the effect of static two-way radio frequency heating;
Fig. 6 is a schematic diagram of the effect of radio frequency rotary heating;
Fig. 7 is a schematic diagram of the effect of combined microwave-radio frequency rotary heating at the initial stage;
Fig. 8 is a schematic diagram of the effect after the microwave radio frequency rotation cooperates with the heating;
Fig. 9 is a schematic flow chart of the global irradiation thermotherapy method according to an embodiment of the disclosure;
In the drawings: 1, capacitor plate; and 2, microwave magnetron. 12

### DETAILED DESCRIPTION OF EMBODIMENTS

The preferred embodiments of the present disclosure will be described in detail below with reference to the accompanying drawings to make the advantages and features of the present disclosure more easily understood by those skilled in the art, so as to make the protection scope of the present disclosure clear and definite.

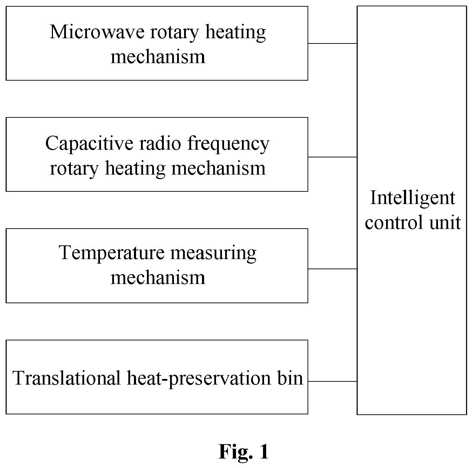

Referring to an embodiment shown in Fig. 1, a global irradiation thermotherapy system based on coordinated rotation of microwave and radio frequency is provided, which mainly includes an intelligent control unit, a microwave rotary heating mechanism, a capacitive radio frequency rotary heating mechanism, a temperature measuring mechanism and a translational heat-preservation bin. The microwave rotary heating mechanism, the capacitive radio frequency rotary heating mechanism, the temperature measuring mechanism and the translational heat-preservation bin are connected to the intelligent control unit.

The capacitive radio frequency rotary heating mechanism rotationally heats up each point in an area of a human body with a lateral radius of 0-13 cm through capacitive radio frequency rotary irradiation to form a circular high-temperature zone. A temperature of the circular high-temperature zone gradually decreases from center to outside. The microwave rotary heating mechanism is configured to to form an annular high-temperature layer with a thickness of 2-4 cm on an inner side of a fat tissue through microwave rotary irradiation, and a temperature of the high-temperature layer decreases from outside to inside. The temperature measurement mechanism is configured to obtain precise temperature data of several key points in an accessible part of a thermotherapy area of the human body via optical-fiber temperature-measuring technology as a reference system, and to accurately measure a temperature of each point in the thermotherapy area based on combination of nuclear magnetic thermal imaging technology. The translational heat-preservation bin is used to carry the human body in and out of an internal space of the microwave rotary heating mechanism and the capacitive radio frequency rotary heating mechanism. The intelligent control unit is used to coordinately control the microwave rotary heating mechanism and the capacitive radio frequency rotary heating mechanism to rotationally irradiate the thermotherapy area to perform deep-shallow layered and complementary diathermy, and to perform precise temperature control based on real-time temperature measurement of the temperature measuring mechanism to achieve uniform and precise hyperthermia throughout the thermotherapy area.

Furthermore, the temperature measurement mechanism includes a nuclear magnetic thermal imaging device, an optical fiber temperature measurement device, and a data processing platform. The nuclear magnetic thermal imaging device is used to establish a temperature field distribution map library of points respectively corresponding to individual temperatures according to the nuclear magnetic resonance scanning data, and to obtain a temperature field distribution map of the thermotherapy area of the human body by scanning. The optical fiber temperature measuring device uses a non-invasive technology to obtain temperatures of several key points in the accessible part of the thermotherapy area as temperature calibration points. The data processing platform is used to calculate the temperature field distribution map scanned by the nuclear magnetic thermal imaging device based on the temperature field distribution map library to obtain a temperature scanning data of each hot point in the thermotherapy area, and is used to calibrate the temperature scanning data of each hot point using temperature data of the temperature calibration points as reference to obtain accurate temperature data of each hot point in the thermotherapy area.

In an embodiment, the optical fiber temperature measurement device is a real-time, on-line, continuous point optical fiber temperature measurement system, which includes a temperature data acquisition unit, a temperature data processing unit, and a sensing optical fiber. The sensing optical fiber is an anti-electromagnetic interference point temperature sensor, on which multiple optical fiber sensors.

A microwave radio frequency coordinated rotation global radiation heat therapy method includes the following steps.

The microwave rotary heating mechanism and the capacitive radio frequency rotary heating mechanism are coordinately controlled by the intelligent control unit to rotationally irradiate the thermotherapy area to perform deep-shallow layered and complementary diathermy, so as to achieve uniform heating of all tissues in the thermotherapy area. The temperature measuring mechanism obtains internal temperature data of the human body via optical-fiber temperature-measuring technology as a reference system, and accurately measures a temperature of each point in the thermotherapy area based on combination of nuclear magnetic thermal imaging technology, so as to achieve precise temperature control and uniform and precise hyperthermia throughout the area.

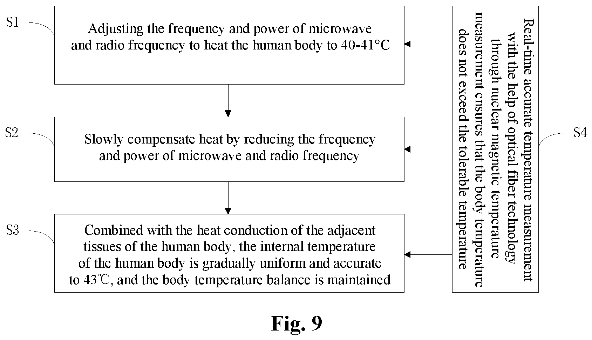

The specific steps of the method will be described below in conjunction with specific embodiments and Fig. 9.

### Embodiment 1

(S1) The intelligent control unit coordinately controls the microwave rotary heating mechanism and the capacitive radio frequency rotary heating mechanism to rotationally irradiate the thermotherapy area to perform deep-shallow layered and complementary diathermy, as well as sets and adjusts a microwave frequency and power and radio frequency and power according to a tolerance of the human body, and guides the translational heat-preservation bin with rotating radiation to move back and forth, so that the thermotherapy area of the human body can be heated quickly and generally evenly to 40-41°C.

In this embodiment, the heating method of microwave combined with capacitive radio frequency rotary irradiation adopts a global irradiation ring for global heating of the human body model. The global irradiation ring is provided with one or more pairs of capacitor plates 1 and one or more microwave magnetrons 2 spaced apart in the circumferential direction. One pair or more pairs of capacitor plates 1 are used for radio frequency diathermy, and the frequency can be selected in the range of 1 MHz-300 MHz. One or more microwave magnetrons 2 are used for microwave heating, and the frequency can be selected from 300 MHz to 3000 MHz. The capacitor plate 1 is set individually, and a power of the capacitor plate 1 is not less than 800 W for heating, such as 1000 W. If there are multiple capacitor plates 1, the total power of the capacitor plates 1 is not less than 800 W for heating. For example, if the number of the capacitor plate 1 is two, a power of one capacitor plate 1 is 400 W and a power of the other capacitor plate 1 is 500 W. The microwave magnetron 2 is set individually, and a power of the microwave magnetron 2 is not less than 600 W for heating, such as 700W. If there are multiple microwave magnetrons 2, a total power is not less than 600 W, for example, if two microwave magnetrons 2 are set, a power of one of the two microwave magnetrons 2 is 400 W and a power of the other is 300 W.

The present disclosure takes advantage of the deep heating of radio frequency and adopts the rotary heating method to uniformly heat each point in an area of the human body with a lateral radius of 0-13 cm, avoiding the problem of overheating of the surface fat layer caused by radio frequency radiation, and enabling the deep layer to be focused to achieve high temperature. Furthermore, this application uses the feature of microwave selective heating and adopts the rotary heating method to avoid the superficial fat layer, to ensure that the fat will not overheat, and to compensate the defect that the 2-6 cm middle layer is not enough due to the insufficient heat of the radio frequency rotary radiation to realize the overall uniform heating. The radio frequency of the capacitive radio frequency rotary heating mechanism is 1 MHz-300 MHz, and the microwave frequency of the microwave rotary heating mechanism is 300 MHz-3000 MHz.

The following describes the global heating mechanism of the present invention with a set of human body model heating effect diagrams in conjunction with this embodiment.

A microwave magnetron 2 adopts 900 MHz, and a first set power is 700W (adjustable). A pair of capacitor plates 1 adopts 40 MHz, and a second set power is 1000 W (adjustable). A circular-sectionhuman body model with a radius of 13 cm simulates a real human body section, where the outermost layer is a fat layer with a thickness of 2 cm, the middle layer of 2-6 cm is the muscle layer, and the other tissue layer is 6-13 cm. Place the human body model in a 37.3°C environment, and fiber optic sensors are arranged from the center to the edge with different depths to measure temperature.

As shown in Fig. 2, the irradiated area facing the microwave magnetron 2 is a local thermotherapy area. The skin temperature outside the fat layer is used as the temperature measurement base point. When the muscle layer inside the fat layer is heated to 43°C, accurate temperature measurement is performed by combination of the nuclear magnetic temperature measurement with the optical-fiber temperature-measuring technology, the temperature rise is as follows: the skin temperature is 37.3°C; the fat layer is about 2 cm thick, and not easy to heat; the temperature of the fat layer is about 38°C; and the temperature of the muscle layer inside the fat layer decreases as the depth increases, from 43°C to 41.5°C. When the heating depth reaches more than 6 cm, the temperature drops sharply to below 38°C. This part of the temperature rise is related to heat conduction.

As shown in Fig. 3, the irradiation area facing the microwave magnetron 2 rotating and irradiating is an annular area. A 4 cm-thick annular high-temperature layer is formed in the inner layer of fat. Take the skin temperature outside the fat layer as the temperature measurement base point. When the muscle layer on the inner side of the fat layer is heated to 43°C, the temperature thereof is accurately measured by nuclear magnetic temperature measurement and optical-fiber temperature-measuring technology. The temperature measurement result is that about 2 cm of the fat layer is still difficult to heat, and the temperature is about 37.5°C. The temperature of the inner muscle layer decreases from 43°C to 41°C with the depth increasing. When the heating depth exceeds 6 cm, the temperature drops below 39°C.

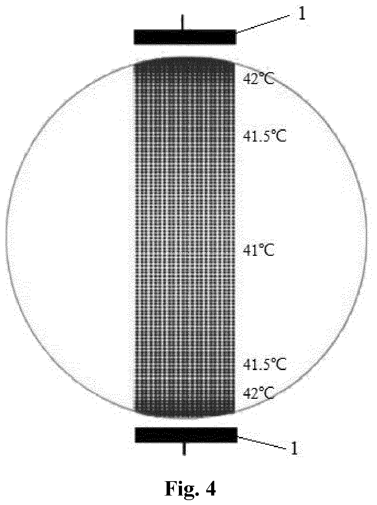

As shown in Fig. 4, the radius of the heating circle section of the human body model is 13 cm. A belt-shaped areaon the human body model between a pair of capacitor plates 1 is an effective heating area. The fat layer dissipates slowly. Take the skin temperature outside the fat layer as the temperature measurement base point. An accurate temperature measurement is carried out through nuclear magnetic temperature measurement and optical-fiber temperature-measuring technology. The temperature measurement result is shown as follows: when the temperature of the fat layer is heated to 42°C, the temperature of the muscle layer on the inner side of the fat layer decreases as the depth increases. The temperature decreased from 42°C to 41°C from the fat layer to the center of the heating circle section.

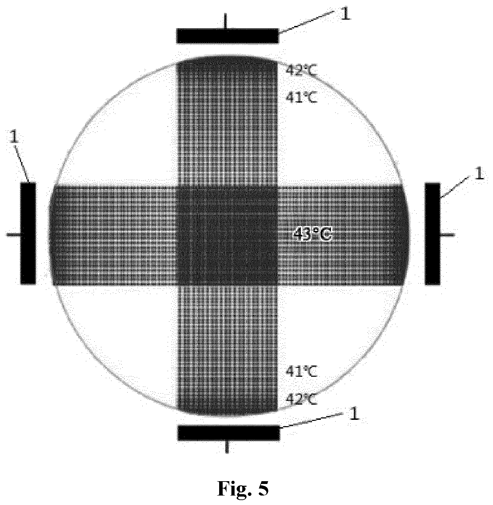

As shown in Fig. 5, two belt-shaped areas form a cross-shaped area, and are the effective heating area. The fat layer dissipates slowly. The skin temperature outside the fat layer is taken as the temperature measurement base point. An accurate temperature measurement is carried out by nuclear magnetic temperature measurement with optical-fiber temperature-measuring technology. The temperature measurement result is described as follows: when the temperature of the fat layer is heated to 42°C, the temperature of the muscle layer on the inner side of the fat layer decreases with the increase in depth, from 42°C to 41.5°C, but the temperature of the cross belt in the central overlapping area rose to 43°C.

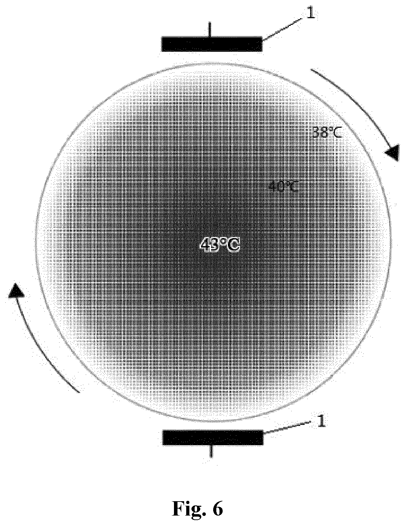

As shown in Fig. 6, when a pair of capacitive pole plates 1 is adopted to rotationally heat the human body model alone, the irradiation area facing the entire heating circle section with a radius of 13 cm. The central temperature is taken as the base point of temperature measurement to perform accurate temperature measurement by nuclear magnetic temperature measurement and optical-fiber temperature-measuring technology. The temperature measurement result is as follows: when the central area is heated to 43°C, the temperature of the heating circle section increases with the increase in depth, which drops from 43°C to 39°C from the central area to the fat layer. The problem of overheating of the fat layer in the state is improved by rotary heating and the fat layer becomes a relatively low-heat area.

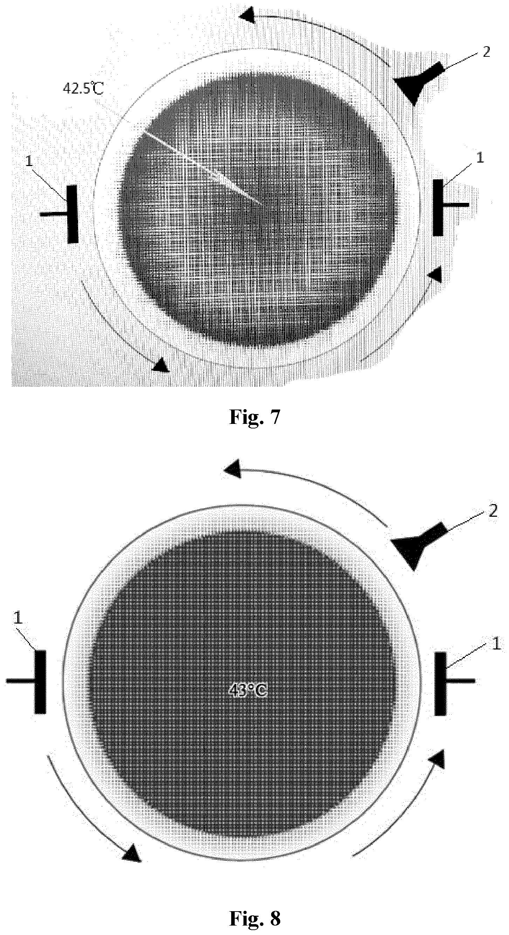

Fig. 7 shows the effect after the microwave rotary irradiation and the radio frequency rotary irradiation synchronized or superimposed on heating, that is, the superimposed diagrams of Fig. 3 and Fig. 6. After synchronizing or superimposing the heating, precise temperature measurement is carried out by nuclear magnetic temperature measurement and optical-fiber temperature-measuring technology. When the temperature in the central area reaches about 42.5°C, about 2 cm of the fat layer is still in the low temperature area, which is at about 38°C; and the temperature of each tissue of the inner side of the fat layer is maintained at a range of 40-42°C, showing a state of uneven temperature distribution.

Fig. 8 shows a heating effect after a global radiation ring continues to slowly supplement heat and conduct heat. The entire heating circle section is slowly supplemented by reducing the power of microwave and radio frequency. After a period of slow supplementation, the temperature is gradually increased while the temperature difference between the tissues is eliminated, and the temperature is slowly increased after a certain period of time. The temperature is finally uniform and maintain a constant temperature after reaching 43°C for a preset time to consolidate the effect of thermotherapy.

(S2) The intelligent control unit slows down the heating speed by adjusting the power of the microwave rotary heating mechanism and the capacitive radio frequency rotary heating mechanism to supplement the heat of the thermotherapy area. It takes no less than 10 minutes for each temperature rise of 1°C.

In this embodiment, the pair of capacitor plates 1 and the microwave magnetron 2 are slowly supplemented with heat at a third set power and a fourth set power, for example, 100W.

(S3) After the temperature of the human body model thermotherapy area rises to 42°C, the intelligent control unit 4 further slows down the heating speed. Combining with the heat conduction between adjacent tissues of the human body model, the internal temperature of the human body model is gradually uniform and accurate to 43°C. The body temperature balance is maintained. The intelligent control unit maintains a relative constant temperature in the thermotherapy area for 30-60 min based on the real-time temperature measurement data under the premise of ensuring that the human body temperature resistance limit is not exceeded.

(S4) The internal temperature data of the human body model obtained via optical-fiber temperature-measuring technology is as reference system to accurately measure a temperature of each point in the thermotherapy area based on combination of nuclear magnetic thermal imaging technology. The temperature measurement data is transmitted to the intelligent control unit to accurately control the human body model heat in real time, such that the temperature of the thermotherapy area does not exceed the tolerance limit. Specifically, the step (S4) is performed through the following steps.

(S401) The nuclear magnetic thermal imaging device scans each hot point in a temperature range (20°C-80°C). A temperature field distribution map library of the hot points respectively corresponding to individual temperatures is established.

The temperature field distribution map library must be composed of thermal imaging data of continuous infinite hot points within a set interval. In actual operation, according to the accuracy requirements, a large amount of thermal imaging data is used to generate a thermal imaging information data map library composed of discontinuous points. The thermal imaging information data map library is taken as a standard reference system to ensure that the temperature distribution map obtained by each nuclear magnetic thermal imaging device has no excessive deviation.

In an embodiment, in the temperature field distribution map library, the thermal imaging map data and temperature value of each hop point are in one-to-one correspondence, which can be represented by two-dimensional coordinates.

(S402) The temperature field distribution map of each point in the thermotherapy area (the composition of important points can be selected according to the accuracy requirements) is obtained through the scanning of the nuclear magnetic thermal imaging device.

The temperature field distribution map includes thermal imaging information and location information of each hot point in the thermotherapy area (important hot points can be selected according to the accuracy requirements). The image information is a computer language containing temperature information, and the location information can use the form of three-dimensional coordinates.

(S403): By the optical fiber temperature measuring device, the temperature of several key points in the accessible part of the thermotherapy area is obtained as the temperature calibration point by using a non-invasive technology.

In an embodiment, the sensing optical fiber is non-invasively guided into tissue cavities in the thermotherapy area such as the gastrointestinal tract, trachea, bladder, uterus and ear chamber from the anus, mouth and nose, urethra, or ear canal, so as to avoid hurts of the human body caused by, for example, introducing the sensing optical fiber into human body by direct minimally invasive surgery.

In an embodiment, the temperature calibration point and its temperature reference include accurate temperature value and location information. The temperature calibration points obtained by the sensing optical fiber have corresponding points in the temperature field distribution map, and the changes of the temperature values of both of them often lead to the same error in the same direction due to the change of obj ective factors.

(S404) The temperature field distribution map obtained in step (S402) is calculated based on the temperature field distribution map library obtained in step (S401) to obtain the temperature scanning data of each point in the thermotherapy area. By taking the temperature data of the temperature calibration points as the reference, the temperature scanning data of each point in the thermotherapy area is calibrated, so as to obtain the accurate temperature data of each point in the thermotherapy area.

For example, the precise temperature of point A on the sensing optical fiber measured by the temperature measuring mechanism 2 is assumed to be 40°C, and the position of the magnetic resonance scan of point A in the temperature field distribution map is point A'. By comparing with the temperature field distribution map library, the scanning temperature is 39.4°C, and the error correction value of the point A and the point A' is +0.6°C. If the position in the temperature field distribution map is point B', and the corresponding scanning temperature is 39.9°C, then the accurate temperature after calibration at this point is 40.5°C.

### Embodiment 2

In this embodiment, the heating method of microwave combined with capacitive radio frequency rotary irradiation still adopts the global irradiation ring to heat the human body. The global irradiation ring is provided with a pair of capacitor plates 1 and microwave magnetrons 2 spaced apart in the circumferential direction. The pair of capacitor plates 1 is used for radio frequency diathermy, and the radio frequency is 5 MHz-200 MHz. The microwave magnetron 2 is used for microwave heating, and the microwave frequency is 300 MHz to 3000 MHz. In step S101, the two pairs of capacitor plates 1 are heated with the first set power and the second set power, respectively, such as 800W, 300W, and the two microwave magnetrons 2 are heated with the third set power and the fourth set power, respectively, such as 500W, 200W, and the temperature of center area and the temperature of the muscle layer under the fat layer are the limit temperature points for temperature calibration. In step S102, the two pairs of capacitor plates 1 and the two microwave magnetrons 2 are both set to a low power, such as 80 W, to slowly compensate heat.

Based on the above heating parameters, steps (S1)-(S4) in Embodiment 1 are performed to implement the global heating of the body. As a consequence, not only the body surface, but also the deep organs can be accurately and easily heated, realizing accurate high heat and blanching throughout the body. The content of steps S1-S4 will not be repeated here.

In the above global heating method, the heating parameters need to be selected by the physician according to human body constitution.

Described above are only preferred embodiments of the present disclosure, which are not intended to limit the scope of the present disclosure. It should be understood that any variations, modifications and replacements made by those skilled in the art without departing from the spirit of the disclosure shall fall within the scope of the disclosure defined by the appended claims.

## Claims

1. A global irradiation thermotherapy system based on coordinated rotation of microwave and radio frequency, comprising:
an intelligent control unit;
a microwave rotary heating mechanism;
a capacitive radio frequency rotary heating mechanism; and
a temperature measuring mechanism;
wherein the microwave rotary heating mechanism, the capacitive radio frequency rotary heating mechanism and the temperature measuring mechanism are connected to the intelligent control unit;
the capacitive radio frequency rotary heating mechanism is configured to rotationally heat each point in an area of a human body with a lateral radius of 0-13 cm through capacitive radio frequency rotary irradiation to form a circular high-temperature zone, wherein a temperature of the circular high-temperature zone decreases from center to outside;
the microwave rotary heating mechanism is configured to form an annular high-temperature layer with a thickness of 2-4 cm on an inner side of a fat tissue through rotary microwave irradiation, and a temperature of the high-temperature layer decreases from outside to inside;
the temperature measuring mechanism is configured to obtain precise temperature data of several key points in an accessible part of a thermotherapy area of the human body via optical-fiber temperature-measuring technology as a reference system, and to accurately measure a temperature of each point in the thermotherapy area based on combination of nuclear magnetic thermal imaging technology; and
the intelligent control unit is configured to coordinately control the microwave rotary heating mechanism and the capacitive radio frequency rotary heating mechanism to rotationally irradiate the thermotherapy area to perform deep-shallow layered and complementary diathermy, and to perform precise temperature control based on real-time temperature measurement of the temperature measuring mechanism to achieve uniform and precise hyperthermia throughout the thermotherapy area.

2. The global irradiation thermotherapy system according to claim 1, **characterized in that** a microwave frequency of the microwave rotary heating mechanism is 300 MHz-3000 MHz, and a radio frequency of the capacitive radio frequency rotary heating mechanism is 1 MHz-300 MHz.

3. The global irradiation thermotherapy system according to claim 1, **characterized in that** the temperature measuring mechanism comprises a nuclear magnetic thermal imaging device, an optical fiber temperature measuring device and a data processing platform;
the nuclear magnetic thermal imaging device is configured to establish a temperature field distribution map library of hot points respectively corresponding to individual temperatures according to nuclear magnetic resonance scanning data, and to obtain a temperature field distribution map of the thermotherapy area of the human body by scanning;
the optical fiber temperature measuring device is configured to obtain temperatures of several key points in the accessible part of the thermotherapy area as temperature calibration points by using a non-invasive technology;
the data processing platform is configured to calculate the temperature field distribution map scanned by the nuclear magnetic thermal imaging device based on the temperature field distribution map library to obtain a temperature scanning data of each hot point in the thermotherapy area, and is configured to calibrate the temperature scanning data of each hot point using temperature data of the temperature calibration points as reference to obtain accurate temperature data of each hot point in the thermotherapy area.

4. The global irradiation thermotherapy system according to claim 3, **characterized in that** the optical fiber temperature measuring device comprises a temperature data acquisition unit, a temperature data processing unit and a sensing optical fiber; and a plurality of optical fiber sensors are provided on the sensing optical fiber.

5. The global irradiation thermotherapy system according to any one of claims 1-4, further comprising:
a translational heat-preservation bin;
wherein the translational heat-preservation bin is configured to carry the human body in and out of an internal space of the microwave rotary heating mechanism and the capacitive radio frequency rotary heating mechanism.

6. A method for measuring a temperature of a thermotherapy area of a human body using the global irradiation thermotherapy system according to claim 3, comprising:
(S401) scanning hot points using the nuclear magnetic thermal imaging device to obtain thermal imaging data, and establishing a temperature field distribution map library of the hot points respectively corresponding to individual temperatures;
(S402) obtaining the temperature field distribution map of the thermotherapy area of the human body by scanning using the nuclear magnetic thermal imaging device;
(S403) obtaining, by the optical fiber temperature measuring device, temperatures of several key points in the accessible part of the thermotherapy area as temperature calibration points by using a non-invasive technology; and
(S404) calculating the temperature field distribution map obtained in step (S402) based on the temperature field distribution map library obtained in step (S401) to obtain a temperature scanning data of each point in the thermotherapy area; and calibrating the temperature scanning data of each point in the thermotherapy area using temperature data of the temperature calibration points as a reference to obtain accurate temperature data of each point in the thermotherapy area.

7. The method according to claim 6, **characterized in that** in step (S401), the temperature field distribution map library comprises thermal imaging data and temperature value of each hot point, and the thermal imaging data and temperature value of each hot point are in one-to-one correspondence.

8. The method according to claim 6, **characterized in that** in step (S404), the temperature data of the temperature calibration points comprises accurate temperature value and location information; and the temperature field distribution map comprises thermal imaging information and location information of each point in the thermotherapy area; and the temperature calibration points have corresponding points in the temperature field distribution map.
